(19) Europäisches Patentamt
European Patent Office
Office européen des brevets

(11) Veröffentlichungsnummer: **0 355 016 B1**

(12) **EUROPÄISCHE PATENTSCHRIFT**

(45) Veröffentlichungstag der Patentschrift: **09.02.94**

(51) Int. Cl.[5]: **C07H 19/00**

(21) Anmeldenummer: **89114833.0**

(22) Anmeldetag: **10.08.89**

# (54) Verfahren zur Herstellung neuer Cytidin-5'-diphosphat-alkanole und -glycerole.

(30) Priorität: **17.08.88 DD 319024**
**02.01.89 DD 324771**
**02.01.89 DD 324772**
**02.01.89 DD 324774**

(43) Veröffentlichungstag der Anmeldung:
**21.02.90 Patentblatt 90/08**

(45) Bekanntmachung des Hinweises auf die Patenterteilung:
**09.02.94 Patentblatt 94/06**

(84) Benannte Vertragsstaaten:
**ES GR**

(56) Entgegenhaltungen:
**GB-A- 2 168 350**
**US-A- 4 291 024**

**CHEMICAL ABSTRACTS, Band 74, 1971, Columbus, OH (US); M. KATES et al., Seite 422, Nr. 112245t&NUM;**

(73) Patentinhaber: **Clarion Pharmaceuticals, Inc.**
**One Harness Court**
**Baltimore, Maryland 21208(US)**

(72) Erfinder: **Brachwitz, Hans**
**Wichertstrasse 9**
**DD- Berlin 1071(DD)**
Erfinder: **Langen, Peter**
**Karowerchaussee 219**
**DD- Berlin 1115(DD)**
Erfinder: **Paltauf, Friedrich**
**St. Peter Hauptstrasse 29b/7**
**A-8036 Graz(AT)**
Erfinder: **Hermetter, Albin**
**Sandgasse 25**
**A-8036 Graz(AT)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**EP 0 355 016 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Cytidin-5'-diphosphatalkanolen und -glycerolen.

Es sind bereits Cytidindiphosphatdialkylglycerole in analoger Weise von M. Kates et al, Can J. Biochem. 49 (1971), 275 hergestellt worden. Diese Verbindungen sind natürlich vorkommend, sie sind beispielsweise Substrate der Phosphatidylinositolsynthase (E.C.2.6.1.67) und reagieren in Gegenwart von Inositol intrazellulär zu Phosphetidylinositol (R.H. Rao , Biochem. Biophys. Acta 348 (1974)306). Die Verbindungen zeigen keinerlei cytostatische Wirksamkeit. Addukte des Cytidinerabinosids (Ara-C), z B.: Ara-C-i-palmitoylglycerol (Chung IL Hong et al. J. Med. Chem., 31, 1988, 1793), sowie Ara-C-acylalkylglycerole (Chung II Hong et el. ebenda, 29, 1986, 20 39) sind ebenfalls bekannt. Sie sind cytostatisch wirksam, wobei die Wirksamkeit auf die Anwesenheit des Ara-C zurückzuführen ist, das selbst ein starkes Cytostatikum darstell: Ara-C-acyl- bzw. alkly-glyceroladdukte lassen sich als Depotform des Ara-C auffassen.

Es konnte nun ein Verfahren zur Herstellung von Cytidin-5'-diphosphatalkanole und -glycerole gefunden werden, die ausgezeichnete cytostatische Wirkung aufweisen.

Gegenstand der Erfindung ist demnach ein Verfahren zur Herstellung von Cytidin-5'-diphosphatalkanolen und -glycerolen der allgemeinen Formel I des Formelblattes, in der R einen gesättigten oder ungesättigten geradkettigen oder verzweigten, unsubstituierten oder durch Halogen, Hydroxy, Alkoxy oder Cyano substituierten aliphatischen Rest mit 6 - 30 C-Atomen oder die Reste

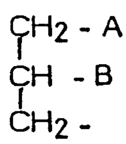 II, 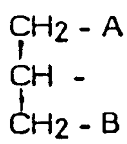 III, 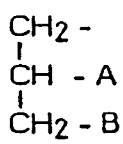 IV,

bedeutet, wobei

- A einen gesättigten oder ungesättigten, geradkettigen oder verzweigten, unsubstituierten oder durch Halogen, Hydroxy, Alkoxy oder Cyano substituierten Alkoxyrest mit 6 - 30 C-Atomen und
- B Wasserstoff, Halogen oder die Reste $-OCH_3$ oder $-O-(CH_2)_nCF_3$, wobei
- n die Zahlen 1 bis 3 bedeutet, durch Umsetzen von Alkylphosphorsäuren bzw. Alkylglycerophosphorsäuren mit Cytidin-5'-phosphat oder einem aktivierten Derivat dieser Verbindung,

das dadurch gekennzeichnet ist, daß man Alkylphosphorsäuren bzw. Alkylglycerophosphorsäuren der allgemeinen Formel V des Formelblattes, in der R die oben angegebene Bedeutung hat, mit Cytidin-5'-phosphat oder einem aktivierten Derivat dieser Verbindung in Gegenwart eines Kondensationsmittels oder einer organischen Base unter wasserfreien Bedingungen umsetzt.

In der Formel I bedeutet R einen gesättigten oder ungesättigten, geradkettigen oder verzweigten aliphatischen Kohlenwasserstoffrest mit 6 - 30 C-Atomen. Vorzugsweise bedeutet R einen gesättigten oder ungesättigten, geradkettigen oder verzweigten Kohlenwasserstoffrest mit 10 - 20 C-Atomen. Beispiele für solche Reste sind Dodecyl-, Tetra-, Penta-, Hexa-, Hepta-, Octa-, Nonadecyl-, Eicosylreste bzw. deren verzweigte Analoge, oder Tetra-, Penta-, Hexa-, Hepta-, Octa-, Nonadecylen oder Eicosylenreste.

Diese Reste können gegebenenfalls euch substituiert sein durch Halogen, beispielsweise Chlor, Brom, Iod oder Fluor, oder durch Hydroxy, Alkoxy oder Cyano.

Weiter kann R die Reste der allgemeinen Formeln II, III oder IV des Formelblattes bedeuten.

In den Formeln II, III und IV bedeutet A einen geradkettigen oder verzweigten oder verzweigten, gesättigten oder ungesättigten Alkoxyrest mit 6 - 30 C-Atomen, vorzugsweise 10 - 20 C-Atomen. Beispiele für solche Alkoxyreste sind Tetra-, Penta-, Hexa-, Hepta-, Octa-, Nonadecyloxy, Eicosyloxyreste, bzw. deren verzweigte Analoge oder Tetra-, Penta-, Hexa-, Hepta-, Octa-, Nona-Dodecadecylenoxy, Eicosylenoxyreste. Diese Reste können gegebenenfalls auch substituiert sein durch Halogen, beispielsweise Chlor, Brom, Iod und Fluor oder Hydroxy, Alkoxy oder Cyano.

B bedeutet Wasserstoff oder Halogen, beispielsweise Chlor, Brom, Iod, Fluor, vorzugsweise Chlor oder die Reste $-OCH_3$ oder $-O-(CH_2)_nCF_3$, wobei n die Zahlen 1 bis 3, vorzugsweise die Zahl 1 bedeutet.

Besonders bevorzugte Einzelverbindungen sind

Cytidin-5'-diphosphat-hexadecanol
Cytidin-5'-diphosphat-dodecanol
Cytidin-5'-diphosphat-2-chlor-2 desoxy-1-0-hexadecylglycerol
Cytidin-5'-diphosphat-1-0-hexadecyl-2-0-(2,2,2-trifluorethyl)glycerol
Cytidin-5'-diphosphat-3-chlor-3 desoxy-1-0-hexadecylglycerol.

Cytidin-5'-diphosphat-1-0-hexadecyl-2-desoxyglycerol
Cytidin-5'-diphosphat-1-0-octadecyl-2-0-methylglycerol

Die Cytidin-5'-diphosphat-alkanole und -glycerole werden hergestellt, indem man Alkylphosphorsäuren bzw. Alkylglycerophosphorsäuren der allgemeinen Formel V des Formelblattes,
in der R die oben angegebene Bedeutung hat, mit Cytidin-5'-phosphat oder einem aktivierten Derivat dieser Verbindung in Gegenwart eines Kondensationsmittels oder einer organischen Base unter wasserfreien Bedingungen umsetzt.

Als aktiviertes Derivat des Cytidin-5'-phosphats kann beispielsweise Cytidin-5'-morpholidophosphat eingesetzt werden. Es ist zweckmäßig, einen der Reaktionspartner in Überschuß einzusetzten, wobei das Molverhältnis Cytidin-5'-phosphat zu Alkylphosphorsäure bzw. Alkylglycerophosphorsäure 1 : 0,5 bis 1 : 2 betragen kann.
Als Kondensationsmittel kann beispielsweise Dicylohexylcarbodiimid verwendet werden.
Als organische Basen werden beispielsweise 4-Dimethylaminopyridin oder Pyridin eingesetzt, wobei bei Verwendung von Pyridin die Umsetzung ohne Zusatz eines organischen Lösungsmittels erfolgen kann.
Gegebenenfalls kann als Lösungsmittel ein inertes organisches Lösungsmittel, beispielsweise Chloroform, eingesetzt werden.
Die Reaktion erfolgt bei Temperaturen von etwa 20 - 60 °C, vorzugsweise 35 - 40 °C.
Nach beendeter Reaktion wird des Produkt aus dem Reaktionsgemisch, beispielsweise durch Extraktion isoliert. Gegebenenfalls können die so erhaltenen Verbindungen, beispielsweise chromatographisch weiter gereinigt werden.

Die Cytidin-5'-diphosphatalkanole und -glycerole zeichnen sich durch hohe cytostatische Wirksamkeit aus.

Zur Bestimmung der cytostatischen Wirkung wurde die antiproliferative Wirkung bei Ehrlich-Ascites Tumorzellen untersucht. Dabei zeigte sich, daß die erfindungsgemäßen Verbindungen ausgezeichnete cytostatische Wirkung aufweisen. Sie können daher allein oder in Mischung mit anderen Wirksubstanzen in Form üblicher galenischer Zubereitungen als Cytostatika oder Virustatika eingesetzt werden.

Die Verbindungen der allgemeinen Formel I des Formelblattes sind zur Verwendung an Menschen bestimmt und können auf übliche Weise, beispielsweise oral oder parenteral verabreicht werden. Vorzugsweise werden sie oral verabreicht, wobei die Tagesdosis etwa 0,05 bis 20 mg/kg Körpergewicht beträgt, vorzugsweise 0,05 - 5 mg/kg Körpergewicht. Der behandelnde Arzt kann jedoch abhängig vom Allgemeinzustand und dem Alter des Patienten, der entsprechenden Verbindung der allgemeinen Formel I des Formelblattes, der Art der Krankheit und der Art der Formulierung auch niedrigere oder höhere Dosen außerhalb dieses Bereiches verschreiben.

Die Verbindungen der Formel I des Formelblattes können allein oder in Verbindung mit anderen pharmazeutisch aktiven Substanzen verabreicht werden, wobei der Gehalt der Verbindungen der allgemeinen Formel I des Formelblattes zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, z. B. pharmazeutisch unbedenklichen Lösungsmitteln, Gelatine, Gummi arabicum, Milchzukker, Stärke, Magnesiumstearat, Talk, pflanzliche Öle, Polyalkylenglykol, Vaseline oder dgl. vor. Die pharmazeutischen Präparate können in fester Form, beispielsweise als Tabletten, Dragees, Suppositorien, Kapseln u. dgl., in halbfester Form beispielsweise als Salben oder in flüssiger Form, beispielsweise als Suspensionen oder Emulsionen vorliegen. Gegenenfalls sind sie sterilisiert und enthalten Hilfsstoffe wie Konservierungs-, Stabilisierungs-und Emulgiermittel, Salz zur Veränderung des osmotischen Drucks u. dgl. Insbesondere können pharmazeutische Präparate die erfindungsgemäßen Verbindungen in Kombination mit anderen therapeutisch wertvollen Stoff enthalten. Mit diesen können die erfindungsgemäßen Verbindungen beispielsweise zusammen mit den oben genannten Hilfs-und/oder Trägerstoffe oder Verdünnungsmitteln zu Kombinationspräparaten formuliert werden.

Beispiel 1:

Cytidin-5'-diphosphat-hexadekanol

Hexadecylphosphat (150 mg, 0,47 mmol) wird viermal in ca. 5 ml Pyridin (über $CaH_2$ getrocknet) aufgenommen und die Lösung jeweils im Vakuum zur Trockne eingeengt.
Cytidin-5'-morpholidophosphat (4-Morpholin-N,N'-dicyclohexyl-carboxamidin-Salz) (404,2 mg, 0,58 mmol) wird viermal mit 4 ml Toluen versetzt und das Gemisch jeweils im Vakuum zur Trockne einrotiert. Danach werden die Reaktionskomponenten vereinigt, erneut mit 5 ml Pyridin einrotiert und in ca. 10 ml Pyridin aufgenommen. Die Lösung wird 5 Tage bei 37 °C unter Ausschluß von Luftfeuchtigkeit gerührt. Anschließend wird das Reaktionsgemisch im Vakuum zur Trockne eingeengt. Zum Rückstand wird Toluen (3 x 5 ml) gegeben, das jeweils im Vakuum abdestilliert wird. Die erhaltene Substanz wird in einem Gemisch aus 15 ml Chloroform-Methanol (1 : 1, v/v) und 7 ml 0,01 N HCl aufgenommen. Die wäßrige Phase wird abgetrennt und dreimal mit etwa 5 ml Chloroform-Methanol extrahiert. Die vereinigten organischen Phasen werden mit wenig Wasser neutral gewaschen und zur Trockene eingeengt. Der Rückstand wird in einem Gemisch aus 45 ml Chloroform, 20 ml Methanol, 9 ml Wasser, 0,3 ml 1 N methanolischen Ammoniak behandelt, die organische Phase wird abgetrennt, nochmals mit 5 ml Wasser extrahiert. Durch Einengen der vereinigten wäßrigen Phasen wird die Verbindung I als Diammoniumsalz erhalten, welches zur weiteren Reinigung aus Chloroform-Aceton umgefällt wird. Die Ausbeute beträgt 144 mg Dünnschichtchloratographie (Merck Fertigplatte Kieselgel 60 F254): $R_f$ 0,31 (Chloroform-Methanol-Eisessig-Wasser, 25 : 15 : 2 : 4, v/v/v/v). UV-Absorption: $\lambda_{max}$ 272,6 nm (Wasser), $\lambda_{max}$ 282 (0,01 N HCl).

Beispiel 2:

Cytidin-5'-diphosphat-1-0-hexadecyl-2-0-(2,2,2-trifluorethyl)glycerol

Eine Mischung von 1-0-Hexadecyl-2-0-(2,2,2-trifluorethyl)glycerophosphorsäure (0,12 g, 0,25 mmol) und Cytidin-5'-morpholidophosphat (4-Morpholin-N,N'-dicyclohexylcarboxamidin-Salz) (0,30 g, 0,44 mmol) wird viermal mit je 5 ml Pyridin (mit $CaH_2$ getrocknet) im Hochvakuum zu Trockne einrotiert. Der Rückstand wird dann erneut in 6 ml Pyridin aufgenommen und die Lösung unter Ausschluß von Feuchtigkeit 9 Tage bei 37 °C gerührt. Danach wird das Reaktionsgemisch im Vakuum zur Trockne eingeengt, der Rückstand mehrfach mit ca. 4 ml Toluen versetzt und erneut zur Trockne gebracht. Die erhaltene Substanz wird in einem Gemisch aus Chloroform-Methanol (1 : 1, v/v, 30 ml) und 0,01 N HCl (15 ml) aufgenommen. Durch tropfenweise Zugabe von 1 N HCl wird die Mischung auf einen pH-Wert von 2 bis 3 eingestellt. Die Chloroform-Methanol-Phase wird abgetrennt, mit Wasser neutral gewaschen und nach Filtrieren eingeengt. Der Rückstand wird in einem Gemisch aus Chloroform (13 ml), Methanol-Wasser (2 : 1, v/v, 10 ml) 1 N methanol. $NH_3$ zweimal mit dem genannten Methanol-Wassermethanol. $NH_3$-Gemisch (ca. je 8 ml) extrahiert. Die vereinigten wäßrigen Phasen werden eingeengt. Der Rückstand wird mit wenig Methanol zur Kristallisation gebracht und anschließend mit trocknem Aceton mehrfach ausgewaschen. Nach dem Trocknen werden 30 mg des Diammoniumsalzes von I als chromatographisch einheitliche Substanz erhalten. Dünnschichtchromatographie (Fertigplatte Kieselgel 60, Merck): Rf 0,39 (n-Propanol/25 %ig, wäßr. $NH_3$/Wasser, 8 : 2 : 1, v/v/v; Rf 0,30 (Chloroform/Methanol/Eisessig/Wasser, 25 : 15 : 2 : 4, v/v/v/v). UV-Absorption: $\lambda_{max}$ 275,5 nm (Wasser); $\lambda_{max}$ 282 nm (0,01 N HCl).

Fest atom bombardment mass spectrometry:

m/z 783 (M)

m/z 558 ($C_{16}H_{33}OCH_2$-CH($OCH_2CF_3$)$CH_2$-O-P(OH)(O)-O-P(OH)(O)-OH),

m/z 540 ($C_{16}H_{33}OCH_2$-CH($OCH_2CF_3$)$CH_2$-O-P(OH)(O)-OP(O)(O)-),

m/z 477 ($C_{16}H_{33}OCH_2$-CH($OCH_2CF_3$)$CH_2$-OP(OH)(O)-O-),

m/z 384 (M-2H($C_{16}H_{33}OCH_2$-CH($OCH_2CF_3$)$CH_2$-0-))

Beispiel 3:

Cytidin-5'-diphosphat-3-chlor-3-desoxy-1-0-hexadecylglycerol

Zu einer Lösung von 0,664 g (1,6 mmol) 3-Chlor-3-desoxy-1-0-hexadecylglycero-2-phosphorsäure und 0,77 g (6,3 mmol) 4-Dimethylaminopyridin in 10 ml getrocknetem Chloroform werden 0,882 (1,3 mmol) Cytidin-5'-morpholidophosphat (4-Morphoiin-N,N',-dicyclohexylcarboxamidin-Salz) gegeben. Die Mischung

wird in einem verschlossenem Gefäß bei 35 °C unter Ausschluß von Feuchtigkeit 5 Tage gerührt. Danach wird das Reaktionsgemisch in Chloroform-Methanol-Wasser (pH-Wert 2) aufgenommen, die organische Phase mit Wasser gewaschen und im Vakuum einrotiert. Der Rückstand wird in Chloroform-Pyridin-Ameisensäure (50 : 30 : 7, v/v/v) aufgenommen und an 30 g Kieselgel 60 /Merck, 0,040 -0,063 mm) säulenchromatographisch getrennt. Die Elution erfolgt zunächst mit 270 ml des gleichen Lösungsmittels, danach mit Chloroform-Methanol (9 : 1, 340 ml; 8 : 2, 300 ml; jeweils v/v).
Die Fraktionen 31 - 55 (Fraktionsgröße 20 ml) werden im Vakuum zur Trockne eingeengt. Der erhaltene Rückstand wird in einem Gemisch aus 28 ml Chloroform-Methanol (1 : 1, v/v) und 9 ml 0,01 N HCl aufgenommen und die Mischung mit 1 N HCl auf den pH-Wert von 2 eingestellt. Die Phasen werden getrennt, die organische Phase wird nochmals mit 5 ml Chloroform-Methanol (1 : 1, v/v) behandelt. Die vereinigten organischen Phasen werden unter Zusatz von methanolischem Ammoniak einrotiert. Das erhaltene Diammoniumsalz der Verbindung I wird durch Behandlung mit Methanol-Diethylether zur Kristallisation gebracht, mit Diethylether gewaschen und getrocknet. Ausbeute: 0,340 g. Dünnschichtchromatographie (Merck Fertigplatte Kieselgel 60 F 254): Rf 0,42 (Chloroform-Methanol-Eisessig-Wasser 50 : 30 : 4 : 8, v/v/v/v); Rf 0,42 (n-Propanol-25 %wäßr.
Ammoniak-Wasser 8 : 2 : 1, v/v/v).
UV-Absorption: $\lambda_{max}$ 281,5 nm (0,01 N HCl).

Beispiel 4:

Cytidin-5'-diphosphat-2-chlor-2-desoxy-1-0-hexadecylglycerol

Eine Mischung, bestehend aus 664 mg 2-Chlor-2-desoxy-1-0-hexadecylglycero-3-phosphorsäure, 882 mg 5'-Cytidinmorpholidophosphat (4-Morpholin-N,N'-dicyclohexylcarboxyamidin-Salz), 770 mg 4-Dimethylaminopyridin, wird unter wasserfreien Bedingungen in 30 ml getrocknetem Chloroform (elkoholfrei) 5 Tage bei 35 °C gerührt, nach beendeter Reaktion wurden zum Reaktionsgemisch Chloroform, Methanol, 0,02 % Salzsäure (je 20 ml) gegeben. Die wäßrige Phase wird mit 25 %iger Salzsäure auf pH 3 gebracht. Die untere Phase wird abgetrennt, mit Wasser gewaschen, getrocknet und im Vakuum zur Trockene eingeengt. Der Rückstand wird in 3 - 5 ml Chloroform/Pyridin/Ameisensäure 50 : 30 : 7, v/v/v, gelöst und in 25 g Kieselgel 60 (Merck, 0,04. - 0,063 mm) chromatographiert. Es wird zunächst mit 200 ml Chloroform/Methanol/Ameisensäure eluiert, danach mit Chloroform/Methanol (jeweils v/v) 9 : 2 (1,1 L), 8 : 2 (0,5 L), 7,5 : 2,5 (0,7 L), 6,5 : 3,5 (0,3 L) und 6 : 4 (2 L). Aus den Cloroform/Methanol-Fraktionen 70 - 96 (Fraktionsgröße 50 ml) werden 200 mg der Verbindung I erhalten. Die Substanz wird anschließend mit Chloroform/Methanol/2,5 n Salzsäure, 2 : 2 : 2, v/v/v, behandelt und die wäßrige Phase auf pH 2,5 eingestellt. Nach Abtrennung der unteren Phase wird die wäßrige Phase nochmals mit Chloroform/Methanol, 2 : 1, v/v, behandelt. Die vereinigten unteren Phasen wurden nach Zugabe von methanolschen Ammoniak einrotiert und so das Ammoniumsalz der Titelverbindung erhalten.
DC (Fertigplatte Kieselgel 60 F 250, Merck), Rf 0,33 ($CHCl_3$/$CH_3OH$, $CH_3$-COOH, $H_2O$, 29 : 18 : 2 : v/v/v/v),
UV-Absorption: $\lambda_{max}$ 275,5 (neutral, $H_2O$), $\lambda_{max}$ 203,6(0,02 N HCl)

Beispiel A

Konzentrationsabhängige Wachstumshemmung von Ehrlich Ascites-Tumorzellen.

| Konzentration (µmolar) | 3 | 10 | 30 | 100 |
|---|---|---|---|---|
| Hemmung (%) | | | | |
| Verbindung nach Bsp. 1 | 12 | 19 | 46 | 31 |
| Verbindung nach Bsp. 2 | 9,5 | 30 | 86 | 100 |
| Verbindung nach Bsp. 3 | 12 | 17 | 29 | 69 |
| Verbindung nach Bsp. 4 | 18 | 38 | 67 | 94 |

Formelblatt

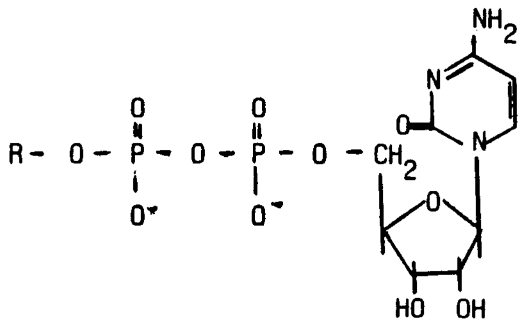

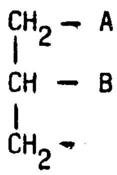

II

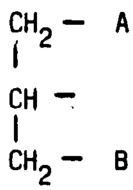

III

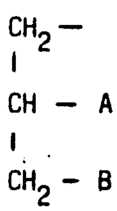

IV

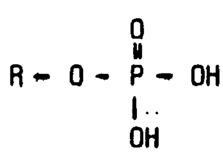

V

## Patentansprüche

1. Verfahren zur Herstellung von Cytidin-5'-diphosphatalkanolen und - glycerolen der allgemeinen Formel I,

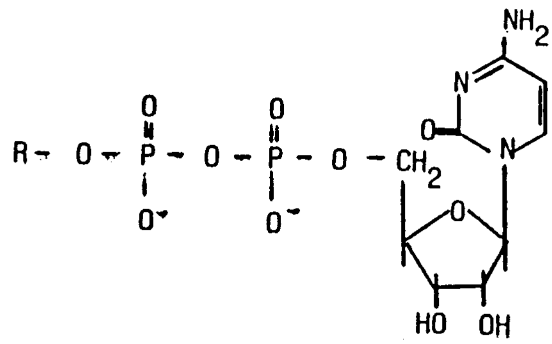

I

in der R einen gesättigten oder ungesättigten, geradkettigen oder verzweigten, unsubstituierten oder durch Halogen, Hydroxy, Alkoxy oder Cyano substituierten aliphatisichen Rest mit 6 - 30 C-Atomen oder die Reste

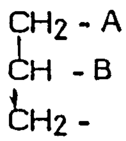 II, 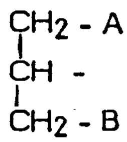 III, 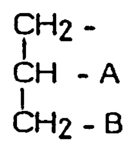 IV,

bedeutet, wobei

A einen gesättigten oder ungesättigten, geradkettigen oder verzweigten, unsubstituierten oder durch Halogen, Hydroxy, Alkoxy oder Cyano substituierten Alkoxyrest mit 6 - 30 C-Atomen und

B Wasserstoff, Halogen oder die Reste $-OCH_3$ oder $-O-(CH_2)_nCF_3$, wobei

n die Zahlen 1 bis 3 bedeutet, durch Umsetzen von Alkylphosphorsäuren bzw. Alkylglycerophosphorsäuren mit Cytidin-5'-phosphat oder einem aktivierten Derivat dieser Verbindung dadurch gekennzeichnet, daß man Alkylphosphorsäuren bzw. Alkylglycerophosphorsäuren der allgemeinen Formel V

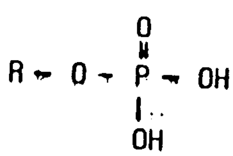

V

in der R die oben angegebene Bedeutung hat, mit Cytidin-5'-phosphat oder einem aktivierten Derivat dieser Verbindung in Gegenwart eines Kondensationsmittels oder einer organischen Base unter wasserfreien Bedingungen umsetzt.

2. Verfahren nach Anspruch 1, dadurch gekennzeichnet, daß als Kondensationsmittel Dicyclohexylcarbodiimid eingesetzt wird.

3. Verfahren nach Anspruch 1, dadurch gekennzeichnet, daß als organische Base Dimethylaminopyridin oder Pyridin eingesetzt wird.

4. Verfahren nach einem der Ansprüche 1 bis 3, dadurch gekennzeichnet, daß die Umsetzung in Gegenwart von Pyridin als organische Base ohne Zusatz eines Lösungsmittels erfolgt.

5. Verfahren nach einem der Ansprüche 1 - 4, dadurch gekennzeichnet, daß die Reaktion bei einer Temperatur von 35 - 40 °C durchgeführt wird.

## Claims

1. Process for the preparation of cytidine 5'-diphosphate alkanols and glycerols having the general formula I,

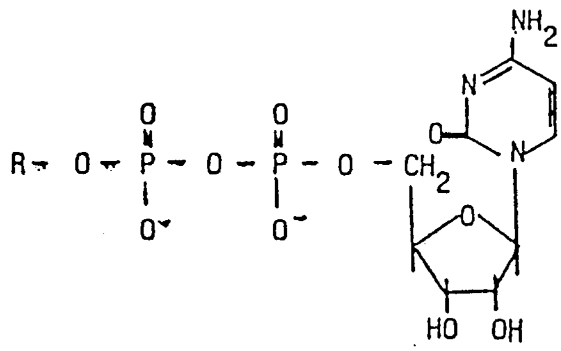

I

in which R represents an aliphatic residue which is saturated or unsaturated, straight-chained or branched, unsubstituted or substituted by halogen, hydroxy, alkoxy or cyano with 6 - 30 C atoms or denotes the residues

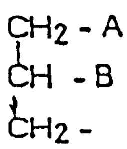 II, 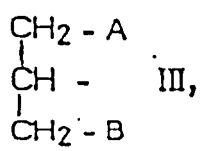 III, 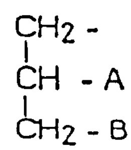 IV,

in which

A denotes an alkoxy residue which is saturated or unsaturated, straight-chained or branched, unsubstituted or substituted by halogen, hydroxy, alkoxy or cyano with 6 - 30 C atoms and

B denotes hydrogen, halogen or the residues $-OCH_3$ or $-O-(CH_2)_nC_3$, in which

n denotes the integers 1 to 3, by reacting alkylphosphoric acids or alkylglycerophosphoric acids with cytidine 5'-phosphate or an activated derivative of this compound, wherein alkylphosphoric acids or alkylglycerophosphoric acids having the general formula V

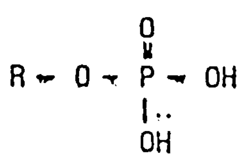

V

in which R has the meaning stated above are reacted with cytidine 5'-phosphate or an activated derivative of this compound in the presence of a condensing agent or an organic base under anhydrous conditions.

2. Process as claimed in claim 1, wherein dicyclohexylcarbodiimide is used as the condensing agent.

3. Process as claimed in claim 1, wherein dimethylaminopyridine or pyridine is used as the organic base.

4. Process as claimed in one of the claims 1 to 3, wherein the reaction is carried out in the presence of pyridine as the organic base without addition of a solvent.

5. Process as claimed in one of the claims 1 - 4, wherein the reaction is carried out at a temperature of 35 - 40 °C.

**Revendications**

1. Procédé pour la préparation de cytidine 5'-diphosphate-alcanols et glycérols de la formule générale I

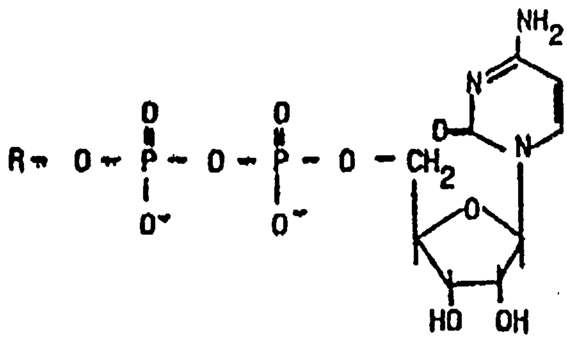

I

dans laquelle R signifie un reste aliphatique saturé ou insaturé, linéaire ou ramifié, non substitué ou substitué par halogène, hydroxy, alcoxy ou cyano avec 6 - 30 atomes C ou les restes

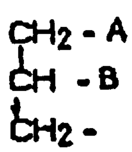

II,

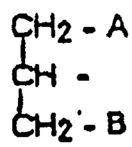

III,

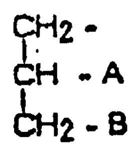

IV,

A signifiant un reste alcoxy saturé ou insaturé, linéaire ou ramifié, non substitué ou substitué par halogène, hydroxy, alcoxy ou cyano avec 6 - 30 atomes C, et

B étant l'hydrogène, l'halogène ou les restes $-OCH_3$ ou $-O-(CH_2)_nCF_3$,

n signifiant les nombres de 1 à 3, par mise en réaction des acides alkylphosphoriques ou des acides alkylglycérophosphoriques avec la cytidine 5'-phosphate ou un dérivé activé de ce composé, caractérisé en ce que l'on met en réaction des acides alkylphosphoriques ou alkylglycérophosphoriques de la formule générale V

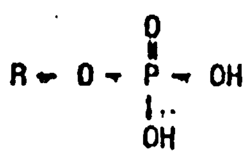

V

dans laquelle R a la signification indiquée ci-dessus, avec la cytidine 5'-phospnate ou un dérivé activé de ce composé en présence d'un agent de condensation ou d'une base organique dans des conditions anhydrées.

2. Procédé selon la revendication 1, caractérisé en ce qu'en tant qu'agent de condensation, on met en oeuvre un dicyclohexylcarbodiimide.

3. Procédé selon la revendication 1, caractérisé en ce qu'en tant que base organique, on met en oeuvre une diméthylaminopyridine ou une pyridine.

4. Procédé selon l'une des revendications 1 à 3, caractérisé en ce que la réaction s'effectue en présence de pyridine en tant que base organique sans addition d'un solvant.

5. Procédé selon l'une des revendications 1 à 4, caractérisé en ce que la réaction s'effectue à une température de 35 - 40 °C.